# EUROPÄISCHE PATENTSCHRIFT

(11) **EP 2 787 987 B1**
(45) Veröffentlichungstag und Bekanntmachung des Hinweises auf die Patenterteilung: **01.06.2016**
(21) Anmeldenummer: 12806375.7
(22) Anmeldetag: 06.12.2012
(51) Int. Cl.: A61K 31/22, A61K 31/366, A61K 31/40, A61K 31/405, A61K 31/44, A61K 31/47, A61K 31/505, A61K 47/12, A61K 47/26, A61K 47/36, A61K 47/38, A61P 3/06

(54) **NEUE PHARMAZEUTISCHE STATIN-ZUSAMMENSETZUNG**
NEW PHARMACEUTICAL STATIN COMPOSITIONS
NOUVEAU COMPOSÉ PHARMACEUTIQUE DE STATINE

(30) Priorität: 08.12.2011 EP 11192673
(43) Veröffentlichungstag der Anmeldung: 15.10.2014
(73) Patentinhaber: Hexal AG, 83607 Holzkirchen (DE)
(72) Erfinder: TAEUBRICH, Theresa, 83607 Holzkirchen (DE); ROTHER, Patrick, 83607 Holzkirchen (DE)
(74) Vertreter: Baumann, Rüdiger Walter
(86) Internationale Anmeldenummer: PCT/EP2012/074600
(87) Internationale Veröffentlichungsnummer: WO 2013/083674

(56) Entgegenhaltungen:
- EP-A1- 2 347 758
- WO-A1-03/082816
- WO-A1-03/092729
- WO-A1-2011/001450
- WO-A2-2005/011638
- WO-A2-2006/054307
- DE-A1- 10 222 326

## Beschreibung

Die Erfindung betrifft eine neue pharmazeutische Zusammensetzung umfassend mindestens eine pharmazeutisch wirksame Substanz der biopharmazeutischen Klasse II, insbesondere mindestens ein Statin. In der erfindungsgemäßen Zusammensetzung kann ohne Nachteile auf die Stabilität des wenigstens einen Wirkstoffs auf antioxidativ wirksame Substanzen verzichtet werden.

Pharmazeutisch wirksame Substanzen der biopharmazeutischen Klasse II (BCS Klasse II) und hierbei insbesondere Statine gelten unter pharmazeutischen Aspekten als instabil und sind besonders anfällig für oxidative Degradation. Um diese zu vermeiden, sind verschiedene Verfahren bzw. Vorgehensweisen bekannt. Hierzu gehören die geeignete Wahl der Darreichungsform, die Formulierung mit geeigneten Hilfsstoffen sowie die Auswahl einer geeigneten Verpackung.

Um die oxidative Degradationsrate von Substanzen der biopharmazeutischen Klasse II, insbesondere der Statine zu reduzieren, werden diese zusammen mit antioxidativ wirksamen Substanzen (Antioxidantien) formuliert. Durch die Antioxidantien sowie weitere Hilfsstoffe in der zumeist oralen pharmazeutischen Zusammensetzung wird der Wirkstoff hinreichend stabilisiert.

Nachteilig hierbei ist, dass zur Wirkstoffstabilisierung unter Umständen redundante Maßnahmen ergriffen werden. Beispielsweise weisen die aus dem Stand der Technik bekannten Darreichungsformen eine Hilfsstoffmenge auf, die sich aufgrund ihrer Höhe nachteilig auf Gewicht und Volumen der Darreichungsform sowie die Herstellungskosten auswirken kann. Je höher der Hilfsstoffanteil ist, desto geringer ist zudem die Konzentration des Wirkstoffs in der Formulierung. Geringe Wirkstoffkonzentrationen haben dabei den Effekt, dass der Anteil an Verunreinigungen, wie z.B. Degradationsprodukten des Wirkstoffs, im Verhältnis zum Wirkstoff nachteilig erhöht ist. Daher wird im Allgemeinen eine hohe Wirkstoffkonzentration, d.h. ein geringer Anteil an Hilfsstoffen in der Formulierung angestrebt.

Im Fall der Substanzen der biopharmazeutischen Klasse II, insbesondere der Statine, tritt demgegenüber als wichtiger Aspekt hinzu, dass sich die Löslichkeit bzw. die Auflösungsgeschwindigkeit dieser Substanzen im physiologischen System erhöht, je höher der Anteil an Hilfsstoffen ist, die in der Darreichungsform verwendet werden.

Als Statine im Zusammenhang mit der vorliegenden Erfindung sind pharmazeutisch wirksame Substanzen zu verstehen, die der pharmakologischen Substanzklasse der 3-Hydroxy-3-Methylglutaryl-Coenzym-A-Reduktase-(HMG-CoA-Reduktase -) Inhibitoren angehören. Statine werden hauptsächlich bei Fettstoffwechselstörungen als Cholesterinsenker eingesetzt und weisen bezogen auf alle Medikamente, die den Lipidstoffwechsel beeinflussen, die höchste Potenz auf.

Bislang sind Statinmedikamente in Form von Tabletten verfügbar, die eine Beschichtung aufweisen (sogenannte filmbeschichtete Tabletten). Zur Stabilisierung der Statine werden diese zusammen mit antioxidativ wirksamen Substanzen formuliert, die vorwiegend den Kettenterminatoren, Reduktionsmitteln, Radikalfängern oder Komplexbildnern zugeordnet werden können. So enthalten die aus dem Stand der Technik bekannten Formulierungen zumeist organische Säuren (z.B. Zitronensäure, Salicylsäure), Phenole und Phenolether (z.B. Butylhydroxyanisol) zum Schutz des Wirkstoffs vor oxidativer Degradation.

WO 03/092729, DE 102 22 326, WO 2006/054307, EP 2 347 758 und WO 2011/001450 beziehen sich auf Zubereitungen enthaltend Statine und Füll sowie Hilfsstoffe und sehen jeweils die Zugabe antioxidativ wirksamer Substanzen als notwendig an, um eine ausreichende Wirkstoffstabilität zu errreichen.

Die in den im Zusammenhang mit der vorliegenden Erfindung beschriebenen Zusammensetzungen verwendbaren Statine werden anhand ihrer biopharmazeutischen Klasse definiert. Das biopharmazeutische Klassifizierungssystem (BCS) teilt pharmazeutisch wirksame Substanzen hinsichtlich ihrer zu erwartenden Bioverfügbarkeit ein. Die in den erfindungsgemäßen Zusammensetzungen verwendeten Statine sind in die biopharmazeutische Klasse II eingeordnet und weisen eine niedrige Löslichkeit im physiologischen System bei hohem Permeationsvermögen auf. Die Resorption wird durch die Löslichkeit und/oder die Lösungsgeschwindigkeit der Arzneistoffe kontrolliert.

Aufgabe der vorliegenden Erfindung ist es, die Nachteile bekannter pharmazeutischer Zusammensetzungen zu überwinden und eine pharmazeutische Zusammensetzung bereit zu stellen, in welcher der bzw. die Wirkstoff(e) der BCS-Klasse II und hierbei insbesondere ein oder mehrere Statine, in vergleichbarer, d.h. pharmazeutisch annehmbarer, Stabilität und Bioverfügbarkeit/Löslichkeit vorliegen. Gleichzeitig soll die bevorzugt feste pharmazeutische Darreichungsform ein niedriges Gewicht bzw. Volumen aufweisen. Insbesondere ist es Aufgabe der Erfindung, eine pharmazeutische Zusammensetzung, umfassend mindestens einen Wirkstoff der BCS-Klasse II und hierbei insbesondere ein oder mehrere Statine bereitzustellen, in welcher die Art und Menge der Hilfsstoffe sowie das Wirkstoff-Hilfsstoff-Mengen- Verhältnis gegenüber bekannten

Formulierungen derart ausgewählt ist, dass das Verhältnis von Wirkstoffstabilität und Bioverfügbarkeit/Löslichkeit des Wirkstoffs optimiert ist. Weiterhin ist es Aufgabe der Erfindung, ein vereinfachtes und kostengünstiges Verfahren zur Herstellung einer pharmazeutischen Zusammensetzung, umfassend einen oder mehrere Wirkstoff(e) der BCS-Klasse II und hierbei insbesondere ein oder mehrere Statine, bereitzustellen.

Die vorgenannten Aufgaben werden gelöst durch Bereitstellen einer pharmazeutischen Zusammensetzung umfassend oder bestehend aus:
(i) 10 bis 30 Gewichts-%, wenigstens einer pharmazeutisch wirksamen Menge einer pharmazeutisch wirksamen Substanz der biopharmazeutischen Klasse II (BCS Klasse II),
(ii) 30 bis 70 Gewichts-% Laktose-Hydrat,
(iii) 2 bis 15 Gewichts-% mikrokristalline Zellulose,
(iv) 5 bis 25 Gewichts-% einer teilweise wasserlöslichen Stärke,
(v) 0,2 bis 4 Gewichts-% wenigstens eines Alkali oder/und Erdalkalisalzes von Stearinsäure oder/und Stearylfumarsäure.

Erfindungsgemäß ist dabei vorgesehen, dass die pharmazeutisch wirksame Substanz der biopharmazeutischen Klasse II ausgewählt ist aus der Gruppe bestehend aus Statinen, vor allem wasserunlöslichen, oxidativ degradierbaren Statinen und hierbei bevorzugt Cerivastatin, Fluvastatin, Lovastatin, Pitavastatin, Pravastatin, Rosuvastatin, Simvastatin oder Kombinationen daraus. Besonders bevorzugt ist im Zusammenhang mit der vorliegenden Erfindung Simvastatin.

Die Erfindung zeichnet sich des Weiteren dadurch aus, dass die Zusammensetzung keine oxidativ wirksame Substanzen wie Kettenterminatoren, Reduktionsmittel, Radikalfänger und Komplexbildner, wie beispielsweise Butylhydroxyanisol, Zitronensäure und Salicylsäure, ohne die Erfindung hierauf zu beschränken, enthält.

Die mit den entsprechenden aus dem Stand der Technik bekannten Medikamenten vergleichbare Wirkstoffstabilität und -bioverfügbarkeit in der erfindungsgemäßen Zusammensetzung ist überraschend, da die erfindungsgemäße Zusammensetzung frei von Antioxidantien sein kann. Ausgehend von den bisher bekannten Formulierungen mit mindestens einem Wirkstoff der BCS Klasse II, insbesondere mit mindestens einem Statin, war anzunehmen, dass antioxidativ wirksame Substanzen wie Butylhydroxyanisol, Zitronensäure und Salicylsäure zur pharmazeutisch hinreichenden Wirkstoffstabilisierung unverzichtbar sind.

Als weiterer Vorteil der erfindungsgemäßen Zusammensetzung hat sich erwiesen, dass aufgrund der Vermeidung unnötiger und/oder unnötig hoher Anteile pharmazeutischer Hilfsstoffe das Gesamtgewicht der bevorzugt festen pharmazeutischen Zusammensetzung minimiert und folglich der mögliche prozentuale Anteil des Wirkstoffes an der pharmazeutischen Zusammensetzung gegenüber bekannten Formulierungen erhöht werden kann. Aufgrund des verringerten Gewichtes lässt sich so auch eine Verringerung der Größe bzw. des Volumens der Darreichungsform bei gleichzeitig höherer Wirkstoffmenge in der Darreichungsform erzielen. Dies steigert wiederum die Patienten-Compliance.

Bevorzugt liegen die Anteile der Komponenten (i) bis (v) an der pharmazeutischen Zusammensetzung, d.h. der (i) pharmazeutisch wirksamen Substanz der biopharmazeutischen Klasse II (BCS Klasse II), des (ii) Laktose-Hydrats, der (iii) mikrokristallinen Zellulose, der (iv) teilweise wasserlöslichen Stärke und des (v) wenigstens eines Alkali oder/und Erdalkalisalzes von Stearinsäure oder/und Stearylfumarsäure zusammen bei 90 bis 100 Gewichts-%, mehr bevorzugt bei 95 bis 100 Gewichts-%, besonders bevorzugt bei 99 bis 100 Gewichts-% und insbesondere bei 99,8 bis 100 Gewichts-%.

Optional umfasst die Zusammensetzung (vi) wenigstens einen weiteren Zusatzstoff aus der Gruppe bestehend aus Füllstoff, Bindemittel, Fließregulierungsmittel, Sprengmittel und Gleitmittel, wobei der Zusatzstoff (vi) von den Komponenten (ii) bis (v) verschieden ist.

Des Weiteren umfasst die Erfindung ein Verfahren zur Herstellung der pharmazeutischen Zusammensetzung.

Unerwartet wurde festgestellt, dass ein Wirkstoff der BCS Klasse II, insbesondere ein Statin, auf einfache und damit kostengünstige Art und Weise in der erfindungsgemäßen Zusammensetzung formuliert werden kann. Die Wirkstoffstabilität in der erfindungsgemäßen pharmazeutischen Zusammensetzung entspricht der Stabilität, die mit den aus dem Stand der Technik bekannten Statinformulierungen erreicht wird. Zudem zeigt ein in der erfindungsgemäßen Zusammensetzung formulierter Wirkstoff nach Applikation eine Auflösungsgeschwindigkeit, die im physiologischen System eine Bioverfügbarkeit gewährleistet, welche zu den bekannten Statinformulierungen äquivalent ist bzw. diese sogar übertrifft.

Als vorteilhaft hat sich erwiesen, wenn der Anteil der wenigstens einen pharmazeutisch wirksamen Substanz, die wie vorstehend definiert ist, an der pharmazeutischen Zusammensetzung bei 11 bis 25 Gewichts-%, bevorzugt zwischen 11 und 20 Gewichts-%, mehr bevorzugt zwischen 13 und 20 Gewichts-% und besonders bevorzugt zwischen 15 und 17 Gewichts-% beträgt. In den genannten Bereichen ist das Verhältnis von Wirkstoffstabilität und -bioverfügbarkeit in der erfindungsgemäßen Formulierung optimiert, d.h. sowohl die Degradationsrate als auch die Löslichkeit des Wirkstoffs, insbesondere ein Statin wie Simvastatin, liegen im pharmazeutisch akzeptablen Bereich und innerhalb der für die Zulassung als Arzneimittel geltenden Anforderungen.

Die unerwarteten, vorstehend genannten vorteilhaften Eigenschaften der erfindungsgemäßen pharmazeutischen Zusammensetzung sind auf eine spezifische Kombination von - als solchen bekannten - Hilfsstoffen für feste Formulierungen zurückzuführen. Neben den wie vorstehend definierten Bestandteilen (ii) bis (v) können in den für ihre jeweiligen Anteile an der Formulierung angegebenen Grenzen andere dem Fachmann bekannte Zusatz- bzw. Hilfsstoffe (vi) in der erfindungsgemäßen Zusammensetzung enthalten sein. Dem Fachmann bekannte Zusatz- bzw. Hilfsstoffe (vi) können z.B. aus der Gruppe bestehend aus Füllstoff, Bindemittel, Fließregulierungsmittel, Sprengmittel und Gleitmittel ausgewählt sein.

In einer besonders bevorzugten Ausführungsform besteht die erfindungsgemäße pharmazeutische Zusammensetzung aus den Bestandteilen (i) bis (v) wie hierin definiert, d.h. die Zusammensetzung enthält neben diesen Bestandteilen keine weiteren Bestandteile.

Eine vorteilhafte Ausführungsform der pharmazeutischen Zusammensetzung sieht vor, dass diese als Tablette gepresst vorliegt. Ein Gegenstand der vorliegenden Erfindung ist daher eine Tablette, die durch Verpressen der erfindungsgemäßen Zusammensetzung erhältlich ist. Die Tablette kann einen Filmüberzug aufweisen. Eine Tablettenbeschichtung ist jedoch bei der erfindungsgemäßen Tablette nicht notwendig. Unter Kostenaspekten ist daher als eine bevorzugte Ausführungsform der Erfindung die pharmazeutische Zusammensetzung zu einer Tablette verpresst, welche nicht filmbeschichtet ist.

Alternativ zu der Verpressung als Tablette besteht die Möglichkeit, dass die Zusammensetzung in einer Gelatine-Hartkapsel eingefüllt vorliegt oder als Pulver oder Granulat vorliegt. Die erfindungsgemäße Zusammensetzung kann zu anderen, dem Fachmann bekannten Verabreichungsformen weiter verarbeitet werden.

Zur Herstellung der erfindungsgemäßen pharmazeutischen Zusammensetzung wird das Laktose-Hydrat bevorzugt ausgewählt aus wenigstens einem Laktose-Hydrat mit einem molaren Verhältnis von Laktose zu Wasser von zwischen 0,25 : 1 und 4:1. Besonders bevorzugt liegt das Verhältnis von Laktose zu Wasser bei zwischen 0,5 : 1 und 2 : 1, insbesondere ist das Verhältnis von Laktose zu Wasser im Wesentlichen 1 : 1. Das heißt, als Bestandteil (ii) der erfindungsgemäßen Zusammensetzung ist Laktose-Monohydrat besonders bevorzugt. In der erfindungsgemäßen Zusammensetzung erweist es sich als besonders vorteilhaft, Granulac^{®}230 als Laktose-Hydratbestandteil einzusetzen.

Bevorzugt beträgt der Anteil des Laktose-Hydrats an der pharmazeutischen Zusammensetzung 45 bis 70 Gewichts-%, wobei Anteile von 50 bis 65 Gewichts-%, insbesondere 55 bis 62,5 Gewichts-% bevorzugt sind. In einer als besonders bevorzugt angesehenen Ausführungsform beträgt der Anteil an Laktose-Hydrat 57,5 bis 60 Gewichts-%.

Zur Herstellung der erfindungsgemäßen pharmazeutischen Zusammensetzung wird als mikrokristalliner Zellulosebestandteil bevorzugt MCC 90µ eingesetzt, jedoch sind auch andere dem Fachmann bekannte pharmazeutisch zugelassene mikrokristalline Zelluloseprodukte geeignet.

Bevorzugt beträgt der Anteil der mikrokristallinen Zellulose an der pharmazeutischen Zusammensetzung 5 bis 15 Gewichts-%, insbesondere 5 bis 13 Gewichts-%. In einer als besonders bevorzugt angesehenen Ausführungsform beträgt der Anteil an mikrokristalliner Zellulose 6 bis 10 Gewichts-%, wobei hier ein Bereich von 7 bis 8 Gewichts-% als besonders günstig anzusehen ist.

Erfindungswesentlich ist, dass die zur Herstellung der erfindungsgemäßen Zusammensetzung eingesetzte Stärke, wie z.B. Maisstärke, eine partiell wasserlösliche Stärke ist. Methoden zur Erzeugung eines teilweise wasserlöslichen Stärkeprodukts zur pharmazeutischen Verwendung sind dem Fachmann bekannt und können chemische und/oder physikalische Modifikationen von Stärke umfassen. Beispiele für chemische Modifikationen zur Erzeugung der partiellen Wasserlöslichkeit von Stärke sind das Vermischen der Stärke mit chemischen Additiven und/oder Tensiden. In der vorliegenden Erfindung sind Stärkeprodukte bevorzugt, die physikalisch modifiziert sind, um die partielle Wasserlöslichkeit zu erreichen. Beispiele für physikalische Modifikationen zur Erzeugung der partiellen Wasserlöslichkeit von Stärke sind die partielle Pregelatinierung der Stärke. Ein besonders geeignetes partiell wasserlösliches Stärkeprodukt zur Herstellung der erfindungsgemäßen Zusammensetzung ist partiell pregelatinierte (Mais-)Stärke, wie das partiell pregelatinierte Maisstärkeprodukt Starch1500^{®} der Firma Colorcon^{®}.

Bevorzugt beträgt der Anteil der partiell wasserlöslichen Stärke an der pharmazeutischen Zusammensetzung 7 bis 18 Gewichts-%, insbesondere 8 bis 16 Gewichts-% oder 10 bis 15 Gewichts-%. In einer als besonders bevorzugt angesehenen Ausführungsform beträgt der Stärkeanteil 14 bis 15 Gewichts-%.

In der erfindungsgemäßen Zusammensetzung ist das Alkali oder/und Erdalkalisalz von Stearinsäure oder/und Stearylfumarsäure bevorzugt ausgewählt aus Magnesiumstearat oder/und Natriumstearylfumarat.

Bevorzugt beträgt der Anteil des Alkali oder/und Erdalkalisalzes von Stearinsäure oder/und Stearylfumarsäure an der pharmazeutischen Zusammensetzung 0,25 bis 3 Gewichts-%, insbesondere 0,75 bis 2,75 Gewichts-%. In einer als besonders bevorzugt angesehenen Ausführungsform beträgt der Anteil des Alkali oder/und Erdalkalisalzes von Stearinsäure oder/und Stearylfumarsäure 1,0 bis 2,5 Gewichts-%.

Die erfindungsgemäße pharmazeutische Zusammensetzung ist durch einen geringen Wasseranteil gekennzeichnet. So beträgt der Wasseranteil an der pharmazeutischen Zusammensetzung höchstens 5 Gewichts-%, bevorzugt höchstens 4 Gewichts-%, mehr bevorzugt höchstens 3 Gewichts-% oder höchstens 2 Gewichts-%. Erreicht wird der niedrige Wasseranteil durch den Ausschluss von Wasser bei der Herstellung der erfindungsgemäßen Zusammensetzung. Bevorzugte Art der Herstellung der erfindungsgemäßen Zusammensetzung ist eine Trockenkompaktierung oder Trockenbrikettierung einer Mischung, enthaltend die Bestandteile (i) bis (v) und gegebenenfalls (vi).

Als bevorzugte Ausführungsform im Hinblick auf das Verhältnis von Wirkstoffstabilität und -bioverfügbarkeit hat sich eine pharmazeutische Zusammensetzung als vorteilhaft erwiesen, umfassend oder bestehen aus
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 45 bis 70 Gewichts-% Laktose-Hydrat, insbesondere Laktose-Monohydrat,
(iii) 5 bis 13 Gewichts-% mikrokristalline Zellulose,
(iv) 7 bis 18 Gewichts-% einer partiell wasserlöslichen Stärke, bevorzugt partiell pregelatinierte Stärke wie Colorcon^{®}Starch1500^{®},
(v) 0,25 bis 3 Gewichts-% Natriumstearat oder/und Natriumstearylfumarat oder/und Magnesiumstearat oder/und Magnesiumstearylfumarat, und optional (vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-%betragen, und wobei der Zusatzstoff (vi) von den Komponenten (ii) bis (v) verschieden ist, wobei die Bestandteile (ii) bis (vi) wie vorstehend definiert sind.

Eine besonders bevorzugte Ausführungsform der Erfindung ist eine pharmazeutische Zusammensetzung, umfassend oder bestehend aus
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 50 bis 65 Gewichts-% Laktose-Hydrat, insbesondere Laktose-Monohydrat, (iii) 7 bis 16 Gewichts-% mikrokristalline Zellulose,
(iv) 8 bis 15 Gewichts-% einer partiell wasserlöslichen Stärke, bevorzugt partiell pregelatinierte Stärke wie Colorcon^{®}Starch1500^{®},
(v) 0,75 bis 2,75 Gewichts-% Natriumstearat oder/und Natriumstearylfumarat oder/und Magnesiumstearat oder/und Magnesiumstearylfumarat, und optional (vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-% betragen, und wobei der Zusatzstoff (vi) von den Komponenten (ii) bis (v) verschieden ist, wobei die Bestandteile (ii) bis (vi) wie vorstehend definiert sind.

Eine weitere besonders bevorzugte Ausführungsform der Erfindung ist eine pharmazeutische Zusammensetzung, umfassend oder bestehend aus
(i) 15 bis 17 Gewichts-% wenigstens eines Statins, insbesondere Simvastatin, (ii) 57,5 bis 60 Gewichts-% Laktose-Hydrat, insbesondere Laktose-Monohydrat,
(iii) 7 bis 8 Gewichts-% mikrokristalline Zellulose,
(iv) 14 bis 16 Gewichts-% einer partiell wasserlöslichen Stärke, bevorzugt partiell pregelatinierte Stärke wie Colorcon^{®}Starch1500^{®},
(v) 1,0 bis 2,5 Gewichts-% Natriumstearat oder/und Natriumstearylfumarat oder/und Magnesiumstearat oder/und Magnesiumstearylfumarat, und optional (vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-% betragen, und wobei der Zusatzstoff (vi) von den Komponenten (ii) bis (v) verschieden ist, wobei die Bestandteile (ii) bis (vi) wie vorstehend definiert sind.

Die vorstehend beschriebene erfindungsgemäße Zusammensetzung liegt als feste Darreichungsform vor und eignet sich insbesondere zur Weiterverarbeitung als Tablette; sie kann aber auch als Granulat oder Pulver vorliegen. Bevorzugt ist die erfindungsgemäße Zusammensetzung als Tablette verpresst. Die durch Verpressen der erfindungsgemäßen Zusammensetzung erhältliche Tablette kann mit einem Filmüberzug versehen sein. Auf eine solche Oberflächenbeschichtung, wie sie von allen derzeit zugelassenen Statin-Tablettenformulierungen bekannt ist, kann aber vorliegend ohne Auswirkung auf die (Langzeit-)Stabilität des Wirkstoffs zu Gunsten einer kostengünstigen Herstellung verzichtet werden. Daher ist die erfindungsgemäße Tablettenformulierung günstiger Weise frei von einem Filmüberzug.

Die positiven Eigenschaften der erfindungsgemäßen Zusammensetzung können weiter verbessert werden, wenn die Dichte der pharmazeutischen Zusammensetzung in einem Bereich von 0,3 g/ml bis 1 g/ml, bevorzugt 0,5 g/ml bis 0,75 g/ml und besonders bevorzugt zwischen 0,55 g/ml und 0,65 g/ml liegt, oder/und die Zusammensetzung eine Korngrößenverteilung mit den Werten:
Korngröße > 500 µm: <10%,
Korngröße 500 µm bis 250 µm: <45%,
Korngröße 250 µm bis 100 µm: >35%,
Korngröße < 100 µm: <10%
aufweist, wobei die Summe der Prozentanteile aller Korngrößen 100 % beträgt.

Wird die erfindungsgemäße Zusammensetzung als Tablette verpresst, kann die Dichte der Zusammensetzung oder/und die Korngrößenverteilung von den oben angegebenen Werten verschieden sein.

In einer besonders bevorzugten Ausführungsform besteht die erfindungsgemäße pharmazeutische Zusammensetzung aus den Bestandteilen (i) bis (v) wie hierin definiert, d.h. die Zusammensetzung enthält neben diesen Bestandteilen keine weiteren Bestandteile und die Anteile der Komponenten (i) bis (v) an der pharmazeutischen Zusammensetzung betragen zusammen 100 Gewichts-%.

In einer weiteren, gleichfalls von der Erfindung umfassten Ausführungsform besteht die erfindungsgemäße pharmazeutische Zusammensetzung aus den folgenden Bestandteilen:
(i) 10 bis 30 Gewichts-%, wenigstens einer pharmazeutisch wirksamen Substanz der biopharmazeutischen Klasse II (BCS Klasse II), insbesondere ein Statin,
(ii) 30 bis 70 Gewichts-% Laktose-Hydrat,
(iii) 2 bis 15 Gewichts-% mikrokristalline Zellulose,
(iv) 5 bis 25 Gewichts-% einer teilweise wasserlöslichen Stärke,
(v) 0,2 bis 4 Gewichts-% wenigstens eines Alkali oder/und Erdalkalisalzes von Stearinsäure oder/und Stearylfumarsäure,
wobei die Anteile der Komponenten (i) bis (v) an der pharmazeutischen Zusammensetzung zusammen 100 Gewichts-% betragen.

In einer weiteren bevorzugten Ausführungsform besteht die erfindungsgemäße pharmazeutische Zusammensetzung aus den folgenden Bestandteilen:
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 45 bis 70 Gewichts-% Laktose-Hydrat, insbesondere Laktose-Monohydrat, (iii) 5 bis 13 Gewichts-% mikrokristalline Zellulose,
(iv) 7 bis 18 Gewichts-% einer partiell wasserlöslichen Stärke, bevorzugt partiell pregelatinierte Stärke wie Colorcon^{®}Starch1500^{®},
(v) 0,25 bis 3 Gewichts-% Natriumstearat oder/und Natriumstearylfumarat oder/und Magnesiumstearat oder/und Magnesiumstearylfumarat, wobei die Anteile der Komponenten (i) bis (v) an der pharmazeutischen Zusammensetzung zusammen 100 Gewichts-% betragen.

In einer besonders bevorzugten Ausführungsform besteht die erfindungsgemäße pharmazeutische Zusammensetzung aus den folgenden Bestandteilen:
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 50 bis 65 Gewichts-% Laktose-Hydrat, insbesondere Laktose-Monohydrat, (iii) 6 bis 10 Gewichts-% mikrokristalline Zellulose,
(iv) 8 bis 15 Gewichts-% einer partiell wasserlöslichen Stärke, bevorzugt partiell pregelatinierte Stärke wie Colorcon^{®}Starch1500^{®},
(v) 0,75 bis 2,75 Gewichts-% Natriumstearat oder/und Natriumstearylfumarat oder/und Magnesiumstearat oder/und Magnesiumstearylfumarat, wobei die Anteile der Komponenten (i) bis (v) an der pharmazeutischen Zusammensetzung zusammen 100 Gewichts-% betragen.

Eine besonders bevorzugte Ausführungsform der Erfindung ist eine pharmazeutische Zusammensetzung, umfassend
(i) 15 bis 17 Gewichts-% wenigstens eines Statins, insbesondere Simvastatin,
(ii) 57,5 bis 60 Gewichts-% Laktose-Hydrat, insbesondere Laktose-Monohydrat,
(iii) 7 bis 8 Gewichts-% mikrokristalline Zellulose,
(iv) 14 bis 16 Gewichts-% einer partiell wasserlöslichen Stärke, bevorzugt partiell pregelatinierte Stärke wie Colorcon^{®}Starch1500^{®},
(v) 1,5 bis 2,5 Gewichts-% Natriumstearat oder/und Natriumstearylfumarat oder/und Magnesiumstearat oder/und Magnesiumstearylfumarat, wobei die Anteile der Komponenten (i) bis (v) an der pharmazeutischen Zusammensetzung zusammen 100 Gewichts-% betragen

Ein weiterer Gegenstand der Erfindung ist ein Verfahren zur Herstellung einer pharmazeutischen Zusammensetzung wie vorstehend definiert, wobei das Verfahren die folgenden Schritte umfasst:
a.) Mischen von
   (i) 10 bis 30 Gewichts-%, wenigstens einer pharmazeutisch wirksamen Substanz der biopharmazeutischen Klasse II (BCS Klasse II), insbesondere eines Statins,
   (ii) 30 bis 70 Gewichts-% Laktose-Hydrat,
   (iii) 2 bis 15 Gewichts-% mikrokristalliner Zellulose, und
   (iv) 5 bis 25 Gewichts-% einer teilweise wasserlöslichen Stärke,
   und optional (vi) wenigstens einem weiteren Zusatzstoff aus der Gruppe bestehend aus Füllstoff, Bindemittel, Fließregulierungsmittel, Sprengmittel und Gleitmittel,
b.) Kompaktieren der aus Schritt a.) erhältlichen Mischung bei einem Druck von 10 bis 40 bar zu Kompaktierkörpern,
c.) Zerkleinern der aus Schritt b) erhältlichen Kompaktierkörper zu einem Pulver oder Granulat oder einer Mischung daraus bis zur gewünschten Korngröße bzw. Korngrößenverteilung,
d.) Zugeben und Vermischen des Bestandteils (v) 0,2 bis 4 Gewichts-% wenigstens eines Alkali- oder/und Erdalkalisalzes von Stearinsäure oder/und Stearylfumarsäure, zu dem aus Schritt c) erhältlichen Pulver oder Granulat oder einer Mischung daraus, und optional e.) Verpressen der aus Schritt d) erhältlichen Mischung zu Tabletten, wobei die Anteile der Komponenten (i) bis (v) an der aus Schritt d) erhältlichen Zusammensetzung oder an der aus Schritt e) erhältlichen Tablette zusammen 90 bis 100 Gewichts-%, bevorzugt 95 bis 100 Gewichts-%, besonders bevorzugt 99 bis 100 Gewichts-% und insbesondere 99,8 bis 100 Gewichts-%betragen, und wobei der Zusatzstoff (vi) von den Komponenten (ii) bis (v) verschieden ist.

Das Verfahren wird in Schritt b) bevorzugt bei einem Druck von 12,5 bis 27,5 bar besonders bevorzugt bei 15 bis 25 bar, insbesondere bei 18 bis 22 bar durchgeführt, wobei der Schritt b) bevorzugt eine Trockenkompaktierung oder Trockenbrikettierung ist, d.h. die Kompaktierung erfolgt unter Ausschluss von Wasser oder wasserhaltigen Lösungsmitteln.

Weiterhin ist vorgesehen, dass die Schritte a) bis d) bei 15 bis 30 °C, bevorzugt bei 18 bis 25 °C, insbesondere bei Raumtemperatur durchgeführt werden.

Der Granulierungsschritt c) des erfindungsgemäßen Verfahrens wird bevorzugt ohne Lösungsmittel und insbesondere ohne Wasser oder wasserhaltige Lösungsmittel, d.h. in Form einer Trockengranulierung, durchgeführt.

Die im Verfahrensschritt a) eingesetzten Bestandteile (i) bis (iv) und optional (vi) sind wie vorstehend für die erfindungsgemäße Zusammensetzung definiert.

Bevorzugt weisen die im erfindungsgemäßen Verfahren verwendeten Bestandteile nachfolgende Gewichtsprozentanteile auf:
(i) 13 bis 20 Gewichts-% wenigstens eines Statins, insbesondere, Cerivastatin, Fluvastatin, Lovastatin, Pitavastatin, Pravastatin, Rosuvastatin, bevorzugt Simvastatin,
(ii) 45 bis 70 Gewichts-% Laktose-Hydrat, insbesondere Laktose-Monohydrat, (iii) 5 bis 13 Gewichts-% mikrokristalline Zellulose,
(iv) 7 bis 18 Gewichts-% einer partiell wasserlöslichen Stärke, bevorzugt partiell pregelatinierte Stärke wie Colorcon^{®}Starch1500^{®},
(v) 0,25 bis 3 Gewichts-% Natriumstearat oder/und Natriumstearylfumarat oder/und Magnesiumstearat oder/und Magnesiumstearylfumarat, und optional
(vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-%betragen, und wobei der Zusatzstoff (vi) von den Komponenten (ii) bis (v) verschieden ist, wobei die Bestandteile (ii) bis (vi) wie vorstehend definiert sind.

Besonders bevorzugt sind die Gewichtsprozentanteile der im erfindungsgemäßen Verfahren verwendeten Bestandteile wie folgt verteilt:
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 50 bis 65 Gewichts-% Laktose-Hydrat, insbesondere Laktose-Monohydrat, (iii) 6 bis 10 Gewichts-% mikrokristalline Zellulose,
(iv) 8 bis 15 Gewichts-% einer partiell wasserlöslichen Stärke, bevorzugt partiell pregelatinierte Stärke wie Colorcon^{®}Starch1500^{®},
(v) 0,75 bis 2,75 Gewichts-% Natriumstearat oder/und Natriumstearylfumarat oder/und Magnesiumstearat oder/und Magnesiumstearylfumarat, und optional
(vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-% betragen, und wobei der Zusatzstoff (vi) von den Komponenten (ii) bis (v) verschieden ist, wobei die Bestandteile (ii) bis (vi) wie vorstehend definiert sind.

Besonders bevorzugt sind die Gewichtsprozentanteile der im erfindungsgemäßen Verfahren verwendeten Bestandteile wie folgt verteilt:
(i) 15 bis 17 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 57,5 bis 60 Gewichts-% Laktose-Hydrat, insbesondere Laktose-Monohydrat,
(iii) 7 bis 8 Gewichts-% mikrokristalline Zellulose,
(iv) 14 bis 16 Gewichts-% einer partiell wasserlöslichen Stärke, bevorzugt partiell pregelatinierte Stärke wie Colorcon^{®}Starch1500^{®},
(v) 1,0 bis 2,5 Gewichts-% Natriumstearat oder/und Natriumstearylfumarat oder/und Magnesiumstearat oder/und Magnesiumstearylfumarat, und optional (vi) wenigstens einen Zusatzstoff aus der Gruppe bestehend aus Füllstoff, Bindemittel, Fließregulierungsmittel, Sprengmittel und Gleitmittel, wobei die Anteile der Komponenten (i) bis (v) an der pharmazeutischen Zusammensetzung zusammen 90 bis 100 Gewichts-%, bevorzugt 95 bis 100 Gewichts-%, besonders bevorzugt 99 bis 100 Gewichts-% und insbesondere 99,8 bis 100 Gewichts-% betragen, und wobei der Zusatzstoff (vi) von den Komponenten (ii) bis (v) verschieden ist, wobei die Bestandteile (ii) bis (vi) wie vorstehend definiert sind.

Um die wie oben beschriebene Schüttdichte und Korngrößenverteilung zu erhalten ist es vorteilhaft, die Bestandteile (i) bis (iv) und optional (vi) vor dem Schritt a) durch ein Sieb mit einem Durchmesser von 0,5 mm bis 2 mm zu geben. Ein Siebdurchmesser von 1 mm hat sich dabei als besonders geeignet erwiesen. Zusätzlich oder alternativ kann hierfür das aus Schritt c) erhältliche Pulver oder Granulat oder eine Mischung daraus vor dem Schritt d) in dem Fachmann bekannten Verfahren homogenisiert werden.

Die Schritte a) bis d) und gegebenenfalls e) des erfindungsgemäßen Verfahrens werden ohne Lösungsmittelzusatz durchgeführt. Insbesondere wird auf die Zugabe von Wasser oder wasserhaltigen Lösungsmitteln im gesamten Verfahren verzichtet.

Die erfindungsgemäßen Verfahrensschritte a) bis d) sind vorteilhaft derart ausgestaltet, dass die aus Schritt d) erhältliche Zusammensetzung bevorzugt folgende Korngrößenverteilung aufweist:
Korngröße > 500 µm: <10%,
Korngröße 500 µm bis 250 µm: <45%,
Korngröße 250 µm bis 100 µm: >35%,
Korngröße < 100 µm: <10%,
wobei die Summe der Prozentanteile aller Korngrößen 100 % beträgt.

Weiterhin sind die Verfahrensschritte a) bis d) vorteilhaft derart ausgestaltet, dass die aus Schritt d) erhältliche Zusammensetzung bevorzugt eine Schüttdichte in einem Bereich von 0,3 g/ml bis 1 g/ml, bevorzugt 0,5 g/ml bis 0,75 g/ml und besonders bevorzugt zwischen 0,55 g/ml und 0,65 g/ml aufweist.

Die am Ende von Schritt d) des erfindungsgemäßen Verfahrens erhältliche Zusammensetzung ist für eine Weiterverarbeitung zu Tabletten besonders geeignet. Somit umfasst das erfindungsgemäße Verfahren bevorzugt zusätzlich den Schritt e) Verpressen der aus Schritt d) erhältlichen Zusammensetzung zu Tabletten, wobei der Schritt e) ohne Zugabe von Lösungsmitteln wie z.B. Wasser durchgeführt wird.

Es konnte gezeigt werden, dass so hergestellte Tabletten Auflösungsprofile und Wirkstoffstabilitäten aufzeigen, die mit denen der aus dem Stand der Technik bekannten und vermarkteten Statintablettenprodukte vergleichbar sind.

Ein weiterer Gegenstand der Erfindung ist eine pharmazeutische Zusammensetzung und eine Tablette, erhältlich durch ein wie vorstehend beschriebenes Verfahren. Die Tablette weist bevorzugt keine Oberflächenbeschichtung auf.

Ein weiterer Gegenstand der Erfindung ist die Verwendung einer pharmazeutischen Zusammensetzung oder einer Tablette wie hierin definiert, in der Medizin, insbesondere als HMG-CoA-Reduktasehemmer, bevorzugt zur Behandlung von Fettstoffwechselstörungen.

### Ausführungsbeispiele

Nachfolgend wird die Erfindung anhand von Beispielen dargestellt. Die gezeigten Ausführungsbeispiele dienen lediglich zur Verdeutlichung der Erfindung, diese ist jedoch nicht auf die in den Ausführungsbeispielen gezeigten Ausführungsformen beschränkt.

### Beispiel 1: Herstellung der pharmazeutischen Zusammensetzung zur oralen Darreichung gemäß der vorliegenden Erfindung

**Table 1**

| Komponenten der pharmazeutischen Zusammensetzung | Menge in mg | Gewicht-% der Zusammensetzung |
|---|---|---|
| Simvastatin | 40,00 | 16,00 |
| Granulac230^{®} (Laktose-Monohydrat) | 149,50 | 59,80 |
| MCC90µ (Mikrokristalline Zellulose) | 18,00 | 7,20 |
| Starch1500^{®} (partiell wasserlösliche Stärke) | 37,50 | 15,00 |
| Magnesiumstearat | 5,00 | 2,00 |
| Gesamtgewicht der Zusammensetzung | 250,00 | 100,00 |

Alle der in Tabelle 1 aufgeführten Bestandteile außer dem Magnesiumstearat bzw. Natriumstearylfumarat wurden durch ein Sieb mit 1,0 mm Maschenweite gegeben und anschließend miteinander vermischt. Die erhaltene Mischung wurde in einen Walzenkompaktator gegeben und mit 15 bis 25 bar Druck und einer kontinuierlichen Zudosierung zu Schülpen gepresst. Die Schülpen werden durch mehrere Mühlen zur gewünschten Korngröße zerkleinert. Anschließend wird die Masse homogenisiert und das Magnesiumstearat bzw. Natriumstearylfumarat zugemischt. Die daraus erhältliche Zusammensetzung kann zu Tabletten verpresst werden.

Das gesamte Verfahren erfolgte bei Raumtemperatur unter GMP-Bedingungen.

### Beispiel 2: Herstellung der pharmazeutischen Zusammensetzung zur oralen Darreichung gemäß der vorliegenden Erfindung

**Table 2**

| Komponenten der pharmazeutischen Zusammensetzung | Menge in mg | Gewicht-% der Zusammensetzung |
|---|---|---|
| Simvastatin | 40,00 | 20,00 |
| Granulac230^{®} (Laktose-Monohydrat) | 109,0 | 54,50 |
| MCC90µ (Mikrokristalline Zellulose) | 0 | 5,00 |
| Starch1500^{®} (partiell wasserlösliche Stärke) | 10,00 | 18,00 |
| Natriumstearylfumarat | 36,00 | 2,50 |
| | 5,00 | |
| Gesamtgewicht der Zusammensetzung | 200,0 0 | 100,00 |

Alle der in Tabelle 2 aufgeführten Bestandteile außer dem Magnesiumstearat bzw. Natriumstearylfumarat wurden durch ein Sieb mit 1,0 mm Maschenweite gegeben und anschließend miteinander vermischt. Die erhaltene Mischung wurde in einen Walzenkompaktator gegeben und mit 15 bis 25 bar Druck und einer kontinuierlichen Zudosierung zu Schülpen gepresst. Die Schülpen werden durch mehrere Mühlen zur gewünschten Korngröße zerkleinert. Anschließend wird die Masse homogenisiert und das Magnesiumstearat bzw. Natriumstearylfumarat zugemischt. Die daraus erhältliche Zusammensetzung kann zu Tabletten verpresst werden.

Das gesamte Verfahren erfolgte bei Raumtemperatur unter GMP-Bedingungen.

### Beispiel 3: Herstellung der pharmazeutischen Zusammensetzung zur oralen Darreichung gemäß der vorliegenden Erfindung

**Table 3**

| Komponenten der pharmazeutischen Zusammensetzung | Menge in mg | Gewicht-% der Zusammensetzung |
|---|---|---|
| Simvastatin | 40,00 | 11,11 |
| Granulac230^{®} (Laktose-Monohydrat) | 233,60 | 64,89 |
| MCC90µ (Mikrokristalline Zellulose) | 36,00 | 10,00 |
| Starch1500^{®} (partiell wasserlösliche Stärke) | 46,80 | 13,00 |
| Natriumstearylfumarat | 3,60 | 1,00 |
| Gesamtgewicht der Zusammensetzung | 360,00 | 100,00 |

Alle der in Tabelle 3 aufgeführten Bestandteile außer dem Magnesiumstearat bzw. Natriumstearylfumarat wurden durch ein Sieb mit 1,0 mm Maschenweite gegeben und anschließend miteinander vermischt. Die erhaltene Mischung wurde in einen Walzenkompaktator gegeben und mit 15 bis 25 bar Druck und einer kontinuierlichen Zudosierung zu Schülpen gepresst. Die Schülpen werden durch mehrere Mühlen zur gewünschten Korngröße zerkleinert. Anschließend wird die Masse homogenisiert und das Magnesiumstearat bzw. Natriumstearylfumarat zugemischt. Die daraus erhältliche Zusammensetzung kann zu Tabletten verpresst werden.

Das gesamte Verfahren erfolgte bei Raumtemperatur unter GMP-Bedingungen.

### Beispiel 4: Herstellung der pharmazeutischen Zusammensetzung zur oralen Darreichung gemäß der vorliegenden Erfindung

**Table 4**

| Komponenten der pharmazeutischen Zusammensetzung | Menge in mg | Gewicht-% der Zusammensetzung |
|---|---|---|
| Simvastatin | 40,00 | 13,99 |
| Granulac230^{®} (Laktose-Monohydrat) | 171,64 | 60,01 |
| MCC90µ (Mikrokristalline Zellulose) | 37,18 | 13,00 |
| Starch1500^{®} (partiell wasserlösliche Stärke) | 31,46 | 11,00 |
| Natriumstearylfumarat | 5,72 | 2,00 |
| Gesamtgewicht der Zusammensetzung | 286,00 | 100,00 |

Alle der in Tabelle 4 aufgeführten Bestandteile außer dem Magnesiumstearat bzw. Natriumstearylfumarat wurden durch ein Sieb mit 1,0 mm Maschenweite gegeben und anschließend miteinander vermischt. Die erhaltene Mischung wurde in einen Walzenkompaktator gegeben und mit 15 bis 25 bar Druck und einer kontinuierlichen Zudosierung zu Schülpen gepresst. Die Schülpen werden durch mehrere Mühlen zur gewünschten Korngröße zerkleinert. Anschließend wird die Masse homogenisiert und das Magnesiumstearat bzw. Natriumstearylfumarat zugemischt. Die daraus erhältliche Zusammensetzung kann zu Tabletten verpresst werden.

Das gesamte Verfahren erfolgte bei Raumtemperatur unter GMP-Bedingungen.

### Die Erfindung umfasst die nachfolgend aufgeführten Gegenstände:

Eine pharmazeutische Zusammensetzung umfassend oder bestehend aus
(i)10 bis 30 Gewichts-%, wenigstens einer pharmazeutisch wirksamen Menge einer pharmazeutisch wirksamen Substanz, ausgewählt aus der Gruppe bestehend aus Statinen, insbesondere wasserunlöslichen, oxidativ degradierbaren Statinen, bevorzugt Cerivastatin, Fluvastatin, Lovastatin, Pitavastatin, Pravastatin, Rosuvastatin, Simvastatin oder Kombinationen daraus,
(ii) 30 bis 70 Gewichts-% Laktose-Hydrat,
(iii) 2 bis 15 Gewichts-% mikrokristalline Zellulose,
(iv) 5 bis 25 Gewichts-% einer teilweise wasserlöslichen Stärke, und
(v) 0,2 bis 4 Gewichts-% wenigstens eines Alkali oder/und Erdalkalisalzes von Stearinsäure oder/und Stearylfumarsäure, wobei die pharmazeutische Zusammensetzung, keine antioxidativ wirksamen Substanzen wie Kettenterminatoren, Radikalfänger und Komplexbildner enthält.

Die zuvor genannte pharmazeutische Zusammensetzung, wobei der Anteil der Komponenten (i) bis (v) an der pharmazeutischen Zusammensetzung zusammen 100 Gewichts-% beträgt.

Insbesondere wobei die pharmazeutische Zusammensetzung weiterhin umfasst:
(vi) wenigstens einen Zusatzstoff aus der Gruppe bestehend aus Füllstoff, Bindemittel, Fließregulierungsmittel, Sprengmittel und Gleitmittel, wobei der Zusatzstoff (vi) von den Komponenten (ii) bis (v) verschieden ist.

Die zuvor genannte pharmazeutische Zusammensetzung, wobei die pharmazeutisch wirksame Substanz Simvastatin ist.

Die zuvor genannte pharmazeutische Zusammensetzung, wobei der Anteil der wenigstens einen pharmazeutisch wirksamen Substanz an der pharmazeutischen Zusammensetzung 11 bis 20 Gewichts-%, insbesondere 13 bis 20 Gew.-%, bevorzugt 15 bis 17 Gew.-% beträgt.

Die zuvor genannte pharmazeutische Zusammensetzung, wobei das Laktose-Hydrat ein molares Verhältnis von Laktose zu Wasser zwischen 0,25 : 1 und 4 : 1, insbesondere zwischen 0,5 : 1 und 2 : 1, bevorzugt von 1 : 1 aufweist.

Die zuvor genannte pharmazeutische Zusammensetzung, wobei das Laktose-Hydrat Laktose-Monohydrat ist.

Die zuvor genannte pharmazeutische Zusammensetzung, wobei das Laktose-Hydrat Granulac^{®}230 ist.

Die zuvor genannte pharmazeutische Zusammensetzung, wobei der Anteil des Laktose-Hydrats an der pharmazeutischen Zusammensetzung zwischen 45 und 70 Gewichts-%, insbesondere zwischen 50 und 65 Gewichts-% , bevorzugt zwischen 55 und 62,5 Gewichts-% oder 57,5 bis 60 Gewichts-% liegt.

Die zuvor genannte pharmazeutische Zusammensetzung, wobei die mikrokristalline Zellulose MCC 90µ ist.

Die zuvor genannte pharmazeutische Zusammensetzung, wobei der Anteil der mikrokristallinen Zellulose an der pharmazeutischen Zusammensetzung zwischen 5 und 15 Gewichts-%, insbesondere zwischen 5 und 13 Gewichts-%, bevorzugt zwischen 6 und 10 Gewichts-% oder zwischen 7 und 8 Gewichts-% liegt.

Die zuvor genannte pharmazeutische Zusammensetzung, wobei die teilweise wasserlösliche Stärke eine partiell pregelatinierte Stärke, insbesondere eine partiell pregelatinierte Maisstärke, bevorzugt Colorcon^{®} Starch1500^{®}, ist.

Die zuvor genannte pharmazeutische Zusammensetzung, wobei der Anteil der Stärke an der pharmazeutischen Zusammensetzung bei zwischen 7 und 18 Gewichts-%, insbesondere bei zwischen 8 und 16 Gewichts-%, bevorzugt bei zwischen 10 und 15 Gewichts-% oder zwischen 14 und 15 Gewichts-% liegt.

Die zuvor genannte pharmazeutische Zusammensetzung, wobei das Alkali- oder/und Erdalkalisalz von Stearinsäure oder/und Stearylfumarsäure Magnesiumstearat oder/und Natriumstearylfumarat ist.

Die zuvor genannte pharmazeutische Zusammensetzung, wobei der Anteil des Alkali- oder Erdalkalisalzes von Stearinsäure oder Stearylfumarsäure an der pharmazeutischen Zusammensetzung zwischen 0,25 und 3 Gewichts-%, insbesondere zwischen 0,75 und 2,75 Gewichts-% bevorzugt zwischen 1,0 und 2,5 Gewichts-% liegt.

Die zuvor genannte pharmazeutische Zusammensetzung, wobei der Wasseranteil an der pharmazeutischen Zusammensetzung höchstens 5 Gewichts-%, insbesondere höchstens 4 Gewichts-%, bevorzugt höchstens 3 Gewichts-%, insbesondere bevorzugt höchstens 2 Gewichts-% beträgt.

Die zuvor genannte pharmazeutische Zusammensetzung, wobei die pharmazeutische Zusammensetzung umfasst oder besteht aus:
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 45 bis 70 Gewichts-% Laktose-Hydrat, insbesondere Laktose-Monohydrat, (iii) 2 bis 15 Gewichts-% mikrokristalline Zellulose,
(iv) 7 bis 18 Gewichts-% einer zumindest teilweise wasserlöslichen, insbesondere partiell pregelatinierten Stärke, und
(v) 0,25 bis 3 Gewichts-% Natriumstearat oder/und Natriumstearylfumaratoder/und Magnesiumstearat oder/und Magnesiumstearylfumarat, wobei die Anteile der Komponenten (i) bis (v) an der pharmazeutischen Zusammensetzung zusammen 90 bis 100 Gewichts-% betragen.

Die zuvor genannte pharmazeutische Zusammensetzung, wobei die pharmazeutische Zusammensetzung weiterhin umfasst:
(vi) wenigstens einen Zusatzstoff aus der Gruppe bestehend aus Füllstoff, Bindemittel, Fließregulierungsmittel, Sprengmittel und Gleitmittel,wobei der Zusatzstoff (vi) von den Komponenten (ii) bis (v) verschieden ist, insbesondere wobei der Anteil des wenigstens eine Zusatzstoffes an der pharmazeutischen Zusammensetzung zwischen 0 und 10 Gewichts-% beträgt.

Die zuvor genannte pharmazeutische Zusammensetzung, wobei die pharmazeutische Zusammensetzung umfasst oder besteht aus
(i) 13 bis 20 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 50 bis 65 Gewichts-% Laktose-Hydrat, insbesondere Laktose-Monohydrat, (iii) 6 bis 10 Gewichts-% mikrokristalline Zellulose,
(iv) 8 bis 15 Gewichts-% einer teilweise wasserlöslichen, insbesondere partiell pregelatinierten Stärke, und
(v) 0,75 bis 2,75 Gewichts-% Natriumstearat oder/und Natriumstearylfumarat oder/und Magnesiumstearat oder/und Magnesiumstearylfumarat, wobei die Anteile der Komponenten (i) bis (v) an der pharmazeutischen Zusammensetzung zusammen 90 bis 100 Gewichts-% betragen.

Die zuvor genannte pharmazeutische Zusammensetzung, wobei die pharmazeutische Zusammensetzung weiterhin umfasst oder besteht aus:
(vi) wenigstens einen Zusatzstoff aus der Gruppe bestehend aus Füllstoff, Bindemittel, Fließregulierungsmittel, Sprengmittel und Gleitmittel, wobei der Zusatzstoff (vi) von den Komponenten (ii) bis (v) verschieden ist, insbesondere wobei der Anteil des wenigstens eine Zusatzstoffes an der pharmazeutischen Zusammensetzung zwischen 0 und 10 Gewichts-% beträgt.

Die zuvor genannte pharmazeutische Zusammensetzung, wobei die pharmazeutische Zusammensetzung umfasst oder besteht aus :
(i) 15 bis 17 Gewichts-% wenigstens eines Statins, insbesondere Cerivastatin, Fluvastatin, Lovastatin, Pitavastatin, Pravastatin, Rosuvastatin, bevorzugt Simvastatin,
(ii) 57,5 bis 60 Gewichts-% Laktose-Hydrat, insbesondere Laktose-Monohydrat,
(iii) 7 bis 8 Gewichts-% mikrokristalline Zellulose,
(iv) 14 bis 16 Gewichts-% einer teilweise wasserlöslichen, insbesondere partiell pregelatinierten Stärke, und
(v) 1,0 bis 2,5 Gewichts-% Natriumstearat oder/und Natriumstearylfumarat oder/und Magnesiumstearat oder/und Magnesiumstearylfumarat, wobei die Anteile der Komponenten (i) bis (v) an der pharmazeutischen Zusammensetzung zusammen 90 bis 100 Gewichts-% betragen.

Die zuvor genannte pharmazeutische Zusammensetzung, wobei die pharmazeutische Zusammensetzung weiterhin umfasst:
(vi) wenigstens einen Zusatzstoff aus der Gruppe bestehend aus Füllstoff, Bindemittel, Fließregulierungsmittel, Sprengmittel und Gleitmittel, wobei der Zusatzstoff (vi) von den Komponenten (ii) bis (v) verschieden ist, insbesondere wobei der Anteil des wenigstens eine Zusatzstoffes an der pharmazeutischen Zusammensetzung zwischen 0 und 10 Gewichts-% beträgt.

Die zuvor genannte pharmazeutische Zusammensetzung, wobei die Zusammensetzung als Tablette gepresst vorliegt.

Die zuvor genannte pharmazeutische Zusammensetzung, wobei die Tablette keine Oberflächenbeschichtung aufweist.

Die zuvor genannte pharmazeutische Zusammensetzung, wobei die Dichte der pharmazeutischen Zusammensetzung 0,3 g/ml bis 1 g/ml insbesondere 0,5 g/ml bis 0,75 g/ml bevorzugt 0,55 g/ml bis 0,65 g/ml beträgt.

Die zuvor genannte pharmazeutische Zusammensetzung, wobei die pharmazeutische Zusammensetzung als feste pharmazeutische Zusammensetzung ausgebildet ist und eine Korngrößenverteilung mit den Werten
Korngröße > 500 µm: <10%,
Korngröße 500 µm bis 250 µm: <45%,
Korngröße 250 µm bis 100 µm: >35%,
Korngröße < 100 µm: <10%
aufweist,
wobei die Summe der Prozentanteile aller Korngrößen 100 % beträgt.

Ein Verfahren zur Herstellung einer pharmazeutischen Zusammensetzung wie zuvor beschrieben, umfassend die Schritte:
a.) Mischen der wie hierin definierten Bestandteile (i) bis (iv) und optional (vi), b.) Kompaktieren der Mischung bei einem Druck von 10 bis 40 bar zu Kompaktierkörpern,
c.) Zerkleinern der Kompaktierkörper zu einem Pulver, einem Granulat und/oder einer Pulver-Granulat-Mischung,
d.) Zugeben und Vermischen des Bestandteils (v) zu dem Pulver, dem Granulat und/oder der Pulver-Granulat-Mischung.

Das zuvor beschriebene Verfahren, wobei das Verfahren zusätzlich den Schritt umfasst:
e.) Verpressen der in Schritt d) erhaltenen Mischung zu Tabletten.

Das zuvor beschriebene Verfahren, wobei als Schritt b) eine Trockenkompaktierung oder Trockenbrikettierung vorgesehen ist, und wobei die Trockenkompaktierung oder Trockenbrikettierung bei einem Druck von zwischen 12,5 und 27,5 bar, insbesondere von zwischen 15 und 25 bar oder 18 bis 22 bar durchgeführt wird.

Das zuvor beschriebene Verfahren, wobei als Schritt c) eine Trockengranulierung vorgesehen ist.

Das zuvor beschriebene Verfahren, wobei die Schritte a) bis d) bei einer Temperatur von zwischen 15 und 30 °C, insbesondere von zwischen 18 und 25 °C, bevorzugt bei Raumtemperatur durchgeführt werden.

Das zuvor beschriebene Verfahren, wobei die Bestandteile (i) bis (iv) und optional (vi) vor Schritt a) durch ein Sieb mit einer Maschenweite von zwischen 0,5 mm und 2 mm, bevorzugt von 1 mm gegeben werden.

Das zuvor beschriebene Verfahren, wobei das aus Schritt c) erhältliche Pulver oder Granulat oder die Pulver-Granulat- Mischung vor Schritt d) homogenisiert wird.

Das zuvor beschriebene Verfahren, wobei die Schritte a) bis d) ohne Zugabe von Wasser oder wasserhaltigen Lösungsmitteln durchgeführt werden.

Das zuvor beschriebene Verfahren, wobei die aus Schritt d) erhältliche Mischung folgende Korngrößenverteilung aufweist:
Korngröße > 500 µm: <10%,
Korngröße 500 µm bis 250 µm: <45%,
Korngröße 250 µm bis 100 µm: >35%,
Korngröße < 100 µm: <10%,
wobei die Summe der Prozentanteile aller Korngrößen 100 % beträgt.

Das zuvor beschriebene Verfahren, wobei die aus Schritt d) erhältliche Mischung eine Dichte von 0,3 g/ml bis 1 g/ml, insbesondere von 0,5 g/ml bis 0,75 g/ml, bevorzugt von 0,55 g/ml bis 0,65 g/ml aufweist.

Das zuvor beschriebene Verfahren, umfassend den Schritt e) Verpressen der aus Schritt d) erhältlichen Mischung zu Tabletten.

Das zuvor beschriebene Verfahren, wobei Schritt e) ohne Zugabe von Wasser oder wasserhaltigen Lösungsmitteln durchgeführt wird.

Eine pharmazeutische Zusammensetzung, erhältlich in einem Verfahren wie zuvor beschrieben.

Eine pharmazeutische Zusammensetzung, erhältlich in einem Verfahren wie zuvor beschrieben, wobei die pharmazeutische Zusammensetzung in Form einer Tablette vorliegt und die Tablette keine Oberflächenbeschichtung aufweist.

Eine Verwendung der pharmazeutischen Zusammensetzung wie zuvor beschrieben in der Medizin, insbesondere als HMG-CoA-Reduktasehemmer, bevorzugt zur Behandlung von Fettstoffwechsel-störungen.

## Patentansprüche

1. Pharmazeutische Zusammensetzung umfassend oder bestehend aus:
(i) 10 bis 30 Gewichts-% wenigstens einer pharmazeutisch wirksamen Menge einer pharmazeutischen Substanz ausgewählt aus der Gruppe bestehend aus wasserunlöslichen, oxidativ degradierbaren Statinen, ausgewählt aus Cerivastatin, Fluvastatin, Lovastatin, Pitavastatin, Pravastatin, Rosuvastatin, Simvastatin oder Kombinationen daraus,
(ii) 30 bis 70 Gewichts-% Laktose-Hydrat,
(iii) 2 bis 15 Gewichts-% mikrokristalline Zellulose,
(iv) 5 bis 25 Gewichts-% einer teilweise wasserlöslichen Stärke,
(v) 0,2 bis 4 Gewichts-% wenigstens eines Alkali oder/und Erdalkalisalzes von Stearinsäure oder/und Stearylfumarsäure,
wobei die Zusammensetzung keine antioxidativ wirksamen Substanzen ausgewählt aus Kettenterminatoren, Reduktionsmittel, Radikalfänger und Komplexbildner enthält.

2. Pharmazeutische Zusammensetzung nach Anspruch 1, **dadurch gekennzeichnet, dass** der Anteil der wenigstens einen pharmazeutisch wirksamen Substanz an der pharmazeutischen Zusammensetzung 11 bis 20 Gewichts-% oder 13 bis 20 Gewichts-% oder 15 bis 17 Gewichts-% beträgt.

3. Pharmazeutische Zusammensetzung nach einem der vorhergehenden Ansprüche, **dadurch gekennzeichnet, dass** das Laktose-Hydrat ein molares Verhältnis von Laktose zu Wasser zwischen 0,25 : 1 und 4 : 1 oder zwischen 0,5 : 1 und 2 : 1 oder 1 : 1 aufweist und der Anteil des Laktose-Hydrats an der pharmazeutischen Zusammensetzung 45 bis 70 Gewichts-% oder 50 bis 65 Gewichts-% oder 55 bis 62,5 Gewichts-% oder 57,5 bis 60 Gewichts-% beträgt.

4. Pharmazeutische Zusammensetzung nach einem der vorhergehenden Ansprüche, **dadurch gekennzeichnet, dass** der Anteil der mikrokristallinen Zellulose an der pharmazeutischen Zusammensetzung 5 bis 15 Gewichts-% oder 5 bis 13 Gewichts-% oder 6 bis 10 Gewichts-% oder 7 bis 8 Gewichts-% beträgt.

5. Pharmazeutische Zusammensetzung nach einem der vorhergehenden Ansprüche, **dadurch gekennzeichnet, dass** die teilweise wasserlösliche Stärke eine partiell pregelatinierte Stärke, bevorzugt eine partiell pregelatinierte Maisstärke wie Colorcon®Starch1500® ist und/oder **dadurch gekennzeichnet, dass** der Stärkeanteil an der pharmazeutischen Zusammensetzung 7 bis 18 Gewichts-% oder 8 bis 16 Gewichts-% oder 10 bis 15 Gewichts-% oder 14 bis 15 Gewichts-% beträgt.

6. Pharmazeutische Zusammensetzung nach einem der vorhergehenden Ansprüche, **dadurch gekennzeichnet, dass** der Anteil des Alkali- oder Erdalkalisalzes von Stearinsäure oder Stearylfumarsäure an der pharmazeutischen Zusammensetzung 0,25 bis 3 Gewichts-% oder 0,75 bis 2,75 Gewichts-% oder 1,0 bis 2,5 Gewichts-% beträgt.

7. Pharmazeutische Zusammensetzung nach einem der vorhergehenden Ansprüche, **dadurch gekennzeichnet, dass** der Wasseranteil an der pharmazeutischen Zusammensetzung höchstens 5 Gewichts-% oder höchstens 4 Gewichts-% oder höchstens 3 Gewichts-% oder höchstens 2 Gewichts-% beträgt.

8. Pharmazeutische Zusammensetzung nach einem der vorhergehenden Ansprüche umfassend oder bestehend aus:
(i) 13 bis 20 Gewichts-% wenigstens eines Statins ausgewählt aus Cerivastatin, Fluvastatin, Lovastatin, Pitavastatin, Pravastatin, Rosuvastatin, bevorzugt Simvastatin,
(ii) 45 bis 70 Gewichts-% Laktose-Hydrat, insbesondere Laktose-Monohydrat,
(iii) 5 bis 13 Gewichts-% mikrokristalline Zellulose,
(iv) 7 bis 18 Gewichts-% einer teilweise wasserlöslichen Stärke, insbesondere einer partiell pregelatinierten Stärke,
(v) 0,25 bis 3 Gewichts-% Natriumstearat oder/und Natriumstearylfumarat oder/und Magnesiumstearat oder/und Magnesiumstearylfumarat, und optional
(vi) wenigstens einen Zusatzstoff aus der Gruppe bestehend aus Füllstoff, Bindemittel, Fließregulierungsmittel, Sprengmittel und Gleitmittel,
wobei die Anteile der Komponenten (i) bis (v) an der pharmazeutischen Zusammensetzung zusammen 90 bis 100 Gewichts-% betragen, und wobei der Zusatzstoff (vi) von den Komponenten (ii) bis (v) verschieden ist.

9. Pharmazeutische Zusammensetzung nach einem der vorhergehenden Ansprüche umfassend oder bestehend aus:
(i) 13 bis 20 Gewichts-% wenigstens eines Statins ausgewählt aus Cerivastatin, Fluvastatin, Lovastatin, Pitavastatin, Pravastatin, Rosuvastatin, bevorzugt Simvastatin,
(ii) 50 bis 65 Gewichts-% Laktose-Hydrat, insbesondere Laktose-Monohydrat,
(iii) 6 bis 10 Gewichts-% mikrokristalline Zellulose,
(iv) 8 bis 15 Gewichts-% einer teilweise wasserlöslichen Stärke, insbesondere einer partiell pregelatinierten Stärke,
(v) 0,75 bis 2,75 Gewichts-% Natriumstearat oder/und Natriumstearylfumarat oder/und Magnesiumstearat oder/und Magnesiumstearylfumarat, und optional
(vi) wenigstens einen Zusatzstoff aus der Gruppe bestehend aus Füllstoff, Bindemittel, Fließregulierungsmittel, Sprengmittel und Gleitmittel,
wobei die Anteile der Komponenten (i) bis (v) an der pharmazeutischen Zusammensetzung zusammen 90 bis 100 Gewichts-% betragen, und wobei der Zusatzstoff (vi) von den Komponenten (ii) bis (v) verschieden ist.

10. Pharmazeutische Zusammensetzung nach einem der vorhergehenden Ansprüche umfassend oder bestehend aus:
(i) 15 bis 17 Gewichts-% Simvastatin,
(ii) 57,5 bis 60 Gewichts-% Laktose-Hydrat, insbesondere Laktose-Monohydrat,
(iii) 7 bis 8 Gewichts-% mikrokristalline Zellulose,
(iv) 14 bis 16 Gewichts-% einer teilweise wasserlöslichen Stärke, insbesondere einer partiell pregelatinierten Stärke
(v) 1,0 bis 2,5 Gewichts-% Natriumstearat oder/und Natriumstearylfumarat oder/und Magnesiumstearat oder/und Magnesiumstearylfumarat, und optional
(vi) wenigstens einen Zusatzstoff aus der Gruppe bestehend aus Füllstoff, Bindemittel, Fließregulierungsmittel, Sprengmittel und Gleitmittel,
wobei die Anteile der Komponenten (i) bis (v) an der pharmazeutischen Zusammensetzung zusammen 90 bis 100 Gewichts-% betragen, und wobei
der Zusatzstoff (vi) von den Komponenten (ii) bis (v) verschieden ist.

11. Pharmazeutische Zusammensetzung nach einem der vorhergehenden Ansprüche, **dadurch gekennzeichnet, dass** die pharmazeutische Zusammensetzung eine feste pharmazeutische Zusammensetzung ist, deren Dichte 0,3 g/ml bis 1 g/ml oder 0,5 g/ml bis 0,75 g/ml oder 0,55 g/ml bis 0,65 g/ml beträgt, und die eine Korngrößenverteilung mit den Werten:
Korngröße > 500 µm: <10%,
Korngröße 500 µm bis 250 µm: <45%,
Korngröße 250 µm bis 100 µm: >35%,
Korngröße < 100 µm: <10% aufweist,
wobei die Summe der Prozentanteile aller Korngrößen 100 % beträgt.

12. Verfahren zur Herstellung einer pharmazeutischen Zusammensetzung gemäß einem der vorhergehenden Ansprüche, wobei das Verfahren die Schritte umfasst:
a.) Mischen der wie in einem der Ansprüche 1 bis 11 definierten Bestandteile (i) bis (iv) und optional (vi),
b.) Kompaktieren der aus Schritt a.) erhältlichen Mischung bei einem Druck von 10 bis 40 bar zu Kompaktierkörpern,
c.) Zerkleinern der Kompaktierkörper zu einem Pulver oder Granulat oder einer Mischung daraus,
d.) Zugeben und Vermischen des Bestandteils (v) zu dem aus Schritt c.) erhältlichen Pulver oder Granulat oder einer Mischung daraus, und optional
e.) Verpressen der aus Schritt d) erhältlichen Mischung zu Tabletten, wobei die Schritte a) bis d) und optional e) bevorzugt ohne Zugabe von Wasser oder wasserhaltigen Lösungsmitteln durchgeführt werden.

13. Pharmazeutische Zusammensetzung, erhältlich in einem Verfahren gemäß Anspruch 12.

14. Pharmazeutische Zusammensetzung, nach Anspruch 13, **dadurch gekennzeichnet, dass** die pharmazeutische Zusammensetzung in Form einer Tablette vorliegt und die Tablette keine Oberflächenbeschichtung aufweist.

15. Pharmazeutische Zusammensetzung nach einem der Ansprüchen 1 bis 14 zur Verwenung in der Medizin, insbesondere als HMG-CoA Reduktasehemmer, bevorzugt in der Behandlung von Fettstoffwechselstörungen.

## Claims

1. A pharmaceutical composition, comprising or consisting of:
(i) 10 to 30% by weight of at least a pharmaceutically active amount of a pharmaceutical substance selected from the group consisting of water-insoluble oxidatively degradable statins, selected from cerivastatin, fluvastatin, lovastatin, pitavastatin, pravastatin, rosuvastatin, simvastatin or combinations thereof,
(ii) 30 to 70% by weight lactose hydrate,
(iii) 2 to 15% by weight microcrystalline cellulose,
(iv) 5 to 25% by weight of a partially water-soluble starch, and
(v) 0.2 to 4% by weight of at least one alkali or/and alkaline earth salt of stearic acid or/and stearyl fumaric acid,
wherein the composition does not contain any antioxidatively active substances selected from chain terminators, reducing agents, free radical scavengers and complexing agents.

2. The pharmaceutical composition according to claim 1, **characterized in that** the proportion of the at least one pharmaceutically active substance in the pharmaceutical composition is 11 to 20% by weight or 13 to 20% by weight or 15 to 17% by weight.

3. The pharmaceutical composition according to one of the preceding claims, **characterized in that** the lactose hydrate has a molar ratio of lactose to water between 0.25:1 and 4:1 or between 0.5:1 and 2:1 or 1:1, and the proportion of lactose hydrate in the pharmaceutical composition is 45 to 70% by weight or 50 to 65% by weight or 55 to 62.5% by weight or 57.5 to 60% by weight.

4. The pharmaceutical composition according to one of the preceding claims, **characterized in that** the proportion of microcrystalline cellulose in the pharmaceutical composition is 5 to 15% by weight or 5 to 13% by weight or 6 to 10% by weight or 7 to 8% by weight.

5. The pharmaceutical composition according to one of the preceding claims, **characterized in that** the partially water-soluble starch is a partially pregelatinized starch, preferably a partially pregelatinized maize starch such as Colorcon^{®}Starch1500^{®}, and/or **characterized in that** the proportion of starch in the pharmaceutical composition is 7 to 18% by weight or 8 to 16% by weight or 10 to 15% by weight or 14 to 15% by weight.

6. The pharmaceutical composition according to one of the preceding claims, **characterized in that** the proportion of the alkali or alkaline earth salt of stearic acid or stearyl fumaric acid in the pharmaceutical composition is 0.25 to 3% by weight or 0.75 to 2.75% by weight or 1.0 to 2.5% by weight.

7. The pharmaceutical composition according to one of the preceding claims, **characterized in that** the proportion of water in the pharmaceutical composition is at most 5% by weight or at most 4% by weight or at most 3% by weight or at most 2% by weight.

8. The pharmaceutical composition according to one of the preceding claims, comprising or consisting of:
(i) 13 to 20% by weight of at least one statin selected from the group consisting of cerivastatin, fluvastatin, lovastatin, pitavastatin, pravastatin, rosuvastatin, and simvastatin,
(ii) 45 to 70% by weight lactose hydrate, in particular lactose monohydrate,
(iii) 5 to 13% by weight microcrystalline cellulose,
(iv) 7 to 18% by weight of a partially water-soluble starch, in particular a partially pregelatinized starch,
(v) 0.25 to 3% by weight sodium stearate and/or sodium stearyl fumarate and/or magnesium stearate and/or magnesium stearyl fumarate, and optionally
(vi) at least one additive from the group consisting of fillers, binders, flow regulators, disintegrants and lubricants,
wherein the proportions of the components (i) to (v) in the pharmaceutical composition together amount to 90 to 100% by weight, and wherein the additive (vi) is different from the components (ii) to (v).

9. The pharmaceutical composition according to one of the preceding claims, comprising or consisting of:
(i) 13 to 20% by weight of at least one statin selected from the group consisting of cerivastatin, fluvastatin, lovastatin, pitavastatin, pravastatin, rosuvastatin, and simvastatin,
(ii) 50 to 65% by weight lactose hydrate, in particular lactose monohydrate,
(iii) 6 to 10% by weight microcrystalline cellulose,
(iv) 8 to 15% by weight of a partially water-soluble starch, in particular a partially pregelatinized starch,
(v) 0.75 to 2.75% by weight sodium stearate and/or sodium stearyl fumarate and/or magnesium stearate and/or magnesium stearyl fumarate, and optionally
(vi) at least one additive from the group consisting of fillers, binders, flow regulators, disintegrants and lubricants,
wherein the proportions of the components (i) to (v) in the pharmaceutical composition together amount to 90 to 100% by weight, and wherein the additive (vi) is different from the components (ii) to (v).

10. The pharmaceutical composition according to one of the preceding claims, comprising or consisting of:
(i) 15 to 17% by weight simvastatin,
(ii) 57.5 to 60% by weight lactose hydrate, in particular lactose monohydrate,
(iii) 7 to 8% by weight microcrystalline cellulose,
(iv) 14 to 16% by weight of a partially water-soluble starch, in particular a partially pregelatinized starch,
(v) 1.0 to 2.5% by weight sodium stearate and/or sodium stearyl fumarate and/or magnesium stearate and/or magnesium stearyl fumarate, and optionally
(vi) at least one additive from the group consisting of fillers, binders, flow regulators, disintegrants and lubricants,
wherein the proportions of the components (i) to (v) in the pharmaceutical composition together amount to 90 to 100% by weight, and wherein the additive (vi) is different from the components (ii) to (v).

11. The pharmaceutical composition according to one of the preceding claims, **characterized in that** the pharmaceutical composition is a solid pharmaceutical composition, the density of which is 0.3 g/mL to 1 g/mL or 0.5 g/mL to 0.75 g/mL or 0.55 g/mL to 0.65 g/mL, said composition having a grain size distribution with the values:
grain size > 500 µm: < 10%,
grain size 500 µm to 250 µm: < 45%,
grain size 250 µm to 100 µm: > 35%,
grain size < 100 µm: < 10%,
wherein the sum of the percentage amounts of all grain sizes is 100%.

12. A method for producing a pharmaceutical composition according to one of the preceding claims, wherein the method comprises the steps of:
a) mixing the components (i) to (iv) and optionally (vi) as defined in one of claims 1 to 11,
b) compacting the mixture obtainable from step a) at a pressure of 10 to 40 bars to form compacted bodies,
c) crushing the compacted bodies to form a powder or granulate or a mixture thereof,
d) adding the component (v) and mixing it with the powder or granulate obtainable from step c) or with a mixture thereof, and optionally
e) pressing the mixture obtainable from step d) to form tablets,
wherein the steps a) to d) and optionally e) are preferably carried out without the addition of water or aqueous solvents.

13. A pharmaceutical composition obtainable in a method according to claim 12.

14. The pharmaceutical composition according to claim 13, **characterized in that** the pharmaceutical composition is present in the form of a tablet and the tablet has no surface coating.

15. The pharmaceutical composition according to one of claims 1 to 14 for use in medicine, in particular as an HMG-CoA reductase inhibitor, preferably in the treatment of fat metabolism disorders.

## Revendications

1. Composition pharmaceutique comprenant ou composée de :
(i) 10 à 30 % en poids d'au moins une quantité pharmaceutiquement active de substances pharmaceutiques sélectionnées dans le groupe des statines insolubles dans l'eau et dégradables par oxydation sélectionnées parmi la cérivastatine, fluvastatine, lovastatine, pitavastatine, pravastatine, rosuvastatine, simvastatine ou leurs combinaisons,
(ii) 30 à 70 % en poids d'hydrate de lactose,
(iii) 2 à 15 % en poids de cellulose microcristalline,
(iv) 5 à 25 % en poids d'amidon partiellement soluble dans l'eau,
(v) 0,2 à 4 % en poids d'au moins un alcali et/ou sel d'alcali terreux d'acide stéarique ou/et d'acide stéaryle fumarique,
la composition ne contenant pas de substances actives du point de vue de l'antioxydation parmi les terminateurs de chaînes, les agents réducteurs, les capteurs de radicaux et les complexants.

2. Composition pharmaceutique selon la revendication 1, **caractérisée en ce que** la proportion de l'au moins une substance pharmaceutiquement active dans la composition pharmaceutique est de de 11 à 20 % en poids ou 13 à 20 % en poids ou 15 à 17 % en poids.

3. Composition pharmaceutique selon une des revendications précédentes, **caractérisée en ce que** l'hydrate de lactose présente un rapport molaire du lactose à l'eau compris entre 0,25:1 et 4:1 ou entre 0,5:1 1 et 2:1 ou 1:1 et que la proportion d'hydrate de lactose dans la composition pharmaceutique est de 45 à 70 % en poids ou 50 à 65 % en poids ou 55 à 62,5 % en poids ou 57,5 à 60 % en poids.

4. Composition pharmaceutique selon une des revendications précédentes, **caractérisée en ce que** la proportion de cellulose microcristalline dans la composition pharmaceutique est de 5 à 15 % en poids ou 5 à 13 % en poids ou 6 à 10 % en poids ou 7 à 8 % en poids.

5. Composition pharmaceutique selon une des revendications précédentes, **caractérisée en ce que** l'amidon partiellement soluble dans l'eau est un amidon partiellement prégélifié, de préférence un amidon de maïs partiellement prégélifié comme le Colorcon^{®}Starch1500^{®} et/ou **caractérisée en ce que** la proportion d'amidon dans la composition pharmaceutique est de 7 à 18 % en poids ou 8 à 16 % en poids ou 10 à 15 % en poids ou 14 à 15 % en poids.

6. Composition pharmaceutique selon une des revendications précédentes, **caractérisée en ce que** la proportion de sel d'alcali ou d'alcali terreux d'acide stéarique ou d'acide stéaryle fumarique dans la composition pharmaceutique est de 0,25 à 3 % en poids ou 0,75 à 2,75 % en poids ou 1,0 à 2,5 % en poids.

7. Composition pharmaceutique selon une des revendications précédentes, **caractérisée en ce que** la proportion d'eau dans la composition pharmaceutique est au plus de 5 % en poids ou au plus de 4 % en poids ou au plus de 3 % en poids ou au plus de 2 % en poids.

8. Composition pharmaceutique selon une des revendications précédentes, comprenant ou composée de :
(i) 13 à 20 % en poids d'au moins une statine sélectionnée parmi la cérivastatine, fluvastatine, lovastatine, pitavastatine, pravastatine, rosuvastatine, de préférence la simvastatine,
(ii) 45 à 70 % en poids d'hydrate de lactose, en particulier de monohydrate de lactose,
(iii) 5 à 13 % en poids de cellulose multicristalline,
(iv) 7 à 18 % en poids d'un amidon partiellement soluble dans l'eau, en particulier d'un amidon partiellement prégélifié,
(v) 0,25 à 3 % en poids de stéarate de sodium et/ou de stéaryle fumarate de sodium et/ou de stéarate de magnésium et/ou de stéaryle fumarate de magnésium et en option
(vi) au moins un additif du groupe composé d'une matière de remplissage, d'un agent liant, d'un agent de régulation d'écoulement, d'un explosif et d'un agent de glissement,
les proportions des composants (i) à (v) représentant ensemble dans la composition pharmaceutique 90 à 100 % en poids et l'additif (vi) étant différent des composants (ii) à (v).

9. Composition pharmaceutique selon une des revendications précédentes, comprenant ou composée de :
(i) 13 à 20 % en poids d'au moins une statine sélectionnée parmi la cérivastatine, fluvastatine, lovastatine, pitavastatine, pravastatine, rosuvastatine, de préférence la simvastatine,
(ii) 50 à 65 % en poids d'hydrate de lactose, en particulier de monohydrate de lactose,
(iii) 6 à 10 % en poids de cellulose multicristalline,
(iv) 8 à 15 % en poids d'un amidon partiellement soluble dans l'eau, en particulier d'un amidon partiellement prégélifié,
(v) 0,75 à 2,75 % en poids de stéarate de sodium et/ou de stéaryle fumarate de sodium et/ou de stéarate de magnésium et/ou de stéaryle fumarate de magnésium et en option
(vi) au moins un additif du groupe composé d'une matière de remplissage, d'un agent liant, d'un agent de régulation d'écoulement, d'un explosif et d'un agent de glissement,
les proportions des composants (i) à (v) représentant ensemble dans la composition pharmaceutique 90 à 100 % en poids et l'additif (vi) étant différent des composants (ii) à (v).

10. Composition pharmaceutique selon une des revendications précédentes, comprenant ou composée de de :
(i) 15 à 17 % en poids de simvastatine,
(ii) 57,5 à 60 % en poids d'hydrate de lactose, en particulier de monohydrate de lactose,
(iii) 7 à 8 % en poids de cellulose multicristalline,
(iv) 14 à 16 % en poids d'un amidon partiellement soluble dans l'eau, en particulier d'un amidon partiellement prégélifié,
(v) 1,0 à 2,5 % en poids de stéarate de sodium et/ou de stéaryle fumarate de sodium et/ou de stéarate de magnésium et/ou de stéaryle fumarate de magnésium et en option
(vi) au moins un additif du groupe composé d'une matière de remplissage, d'un agent liant, d'un agent de régulation d'écoulement, d'un explosif et d'un agent de glissement,
les proportions des composants (i) à (v) représentant ensemble dans la composition pharmaceutique 90 à 100 % en poids et l'additif (vi) étant différent des composants (ii) à (v).

11. Composition pharmaceutique selon une des revendications précédentes, **caractérisée en ce que** la composition pharmaceutique est une composition pharmaceutique solide dont la densité est de 0,3 g/ml à 1 g/ml ou 0,5 g/ml à 0,75 g/ml ou 0,55 g/ml à 0,65 g/ml et qui présente une répartition de granulométrie présentant les valeurs suivantes :
granulométrie > 500 µm : < 10 %,
granulométrie 500 µm à 250 µm : < 45 %,
granulométrie 250 µm à 100 µm : > 35 %,
granulométrie < 100 µm : < 10 %,
la somme des pourcentages de toutes les granulométries étant de 100 %.

12. Procédé de préparation d'une composition pharmaceutique selon une des revendications précédentes, ce procédé comprenant les étapes suivantes :
a. ) mélange des composants (i) à (iv) tels que définies dans une des revendications 1 à 11 et en option (vi),
b. ) compactage du mélange pouvant être obtenu en étape a.) avec une pression de 10 à 40 bars pour former des corps compactés,
c.) broyage des corps compactés pour obtenir une poudre ou un granulé ou un mélange de ceux-ci,
d. ) ajout et mélange du composant (v) dans la poudre ou le granulé ou leur mélange pouvant être obtenu en étape c.) et en option
e. compression du mélange pouvant être obtenu en étape d) en comprimés, les étapes a) à d) et en option e) étant réalisées de préférence sans ajout d'eau ou de solvant aqueux.

13. Composition pharmaceutique pouvant être obtenue dans un procédé selon la revendication 12.

14. Composition pharmaceutique selon la revendication 13, **caractérisée en ce que** la composition pharmaceutique se présente sous forme d'un comprimé et que le comprimé ne présente pas d'enrobage de surface.

15. Composition pharmaceutique selon une des revendications 1 à 14, destinée à une utilisation en médecine, en particulier comme inhibiteur de réductase HMG-CoA, de préférence dans le traitement des troubles du métabolisme des graisses.
